# EUROPÄISCHE PATENTANMELDUNG

(11) **EP 0 123 971 A2**
(43) Veröffentlichungstag der Anmeldung: **07.11.1984**
(21) Anmeldenummer: 84103926.6
(22) Anmeldetag: 13.07.1981
(51) Int. Cl.: C07D 249/08, A01N 43/64

(54) **Triazolylpropenol-Derivate, Verfahren zu ihrer Herstellung sowie ihre Verwendung als Pflanzenwachstumsregulatoren und Fungizide**

(30) Priorität: 25.07.1980 DE 3028330; 20.03.1981 DE 3111013
(62) Teilanmeldung aus: 81105442.8
(71) Anmelder: BAYER AG, 51368 Leverkusen (DE)
(72) Erfinder: Regel, Erik, Dipl.-Ing., D-5600 Wuppertal 1 (DE); Büchel, Karl Heinz, Prof. Dr., D-5093 Burscheid (DE); Lürssen, Klaus, Dr., D-5060 Bergisch-Glasbach 2 (DE); Frohberger, Paul-Ernst, Dr., D-5090 Leverkusen 1 (DE); Paul, Volker, Dr., D-4770 Soest (DE)

(57) **Zusammenfassung**

Neue Triazolylpenol-Derivate, ein Verfahren zu ihrer Herstellung durch Reduktion der entsprechenden Triazolylpropenon-Derivate, und die Verwendung der erfindungsgemäßen Stoffe als Pflanzenwachstumsregulatoren und Fungizide.

Die bei der Herstellung der Triazolylpropenol-Derivate als Ausgangsstoffe benötigten Triazolylpropenon-Derivate sind ebenfalls neu.

## Beschreibung

Die vorliegende Erfindung betrifft neue Triazolylpropenol-Derivate, ein Verfahren zu ihrer Herstellung sowie ihre Verwendung als Pflanzenwachstumsregulatoren und Fungizide.

Es ist bereits bekannt geworden, daß 4,4-Dimethyl-1-phenyl-2-triazolyl-1-penten-3-ole eine gute fungizide Wirksamkeit besitzen (vgl. DE-A 28 38 847). Die Wirkung dieser Verbindungen ist jedoch, insbesondere bei niedrigen Aufwandmengen und -konzentrationen, nicht immer voll befriedigend. Die pflanzenwachstumsregulierende Wirkung dieser Azol-Derivate ist ebenfalls nicht immer ganz befriedigend.

Es wurden neue Triazolylpropenol-Derivate der Formeln 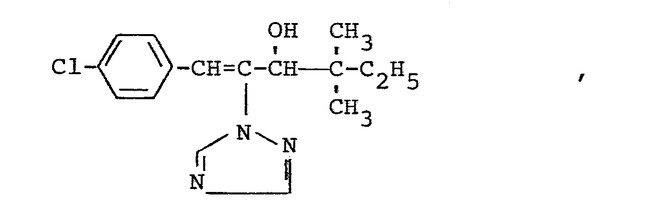 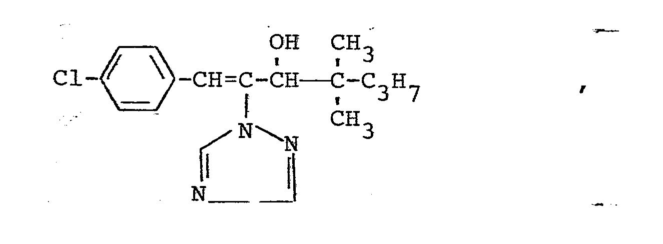 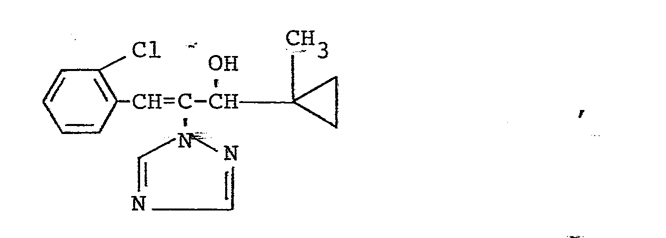 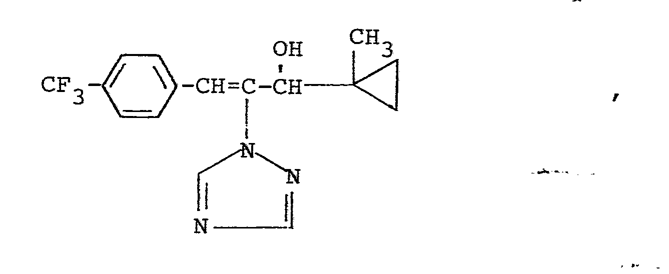 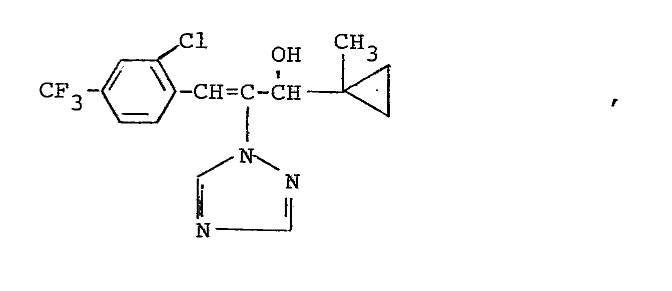 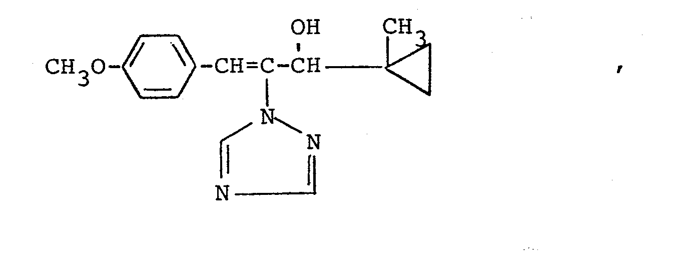 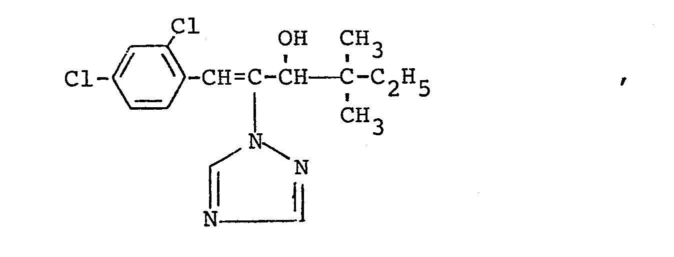 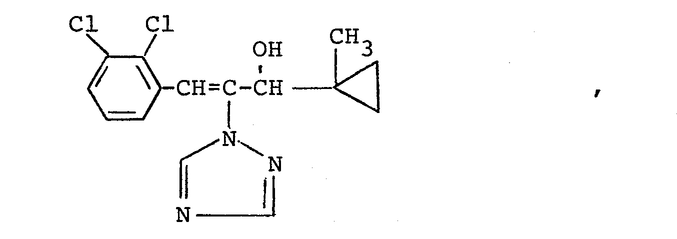 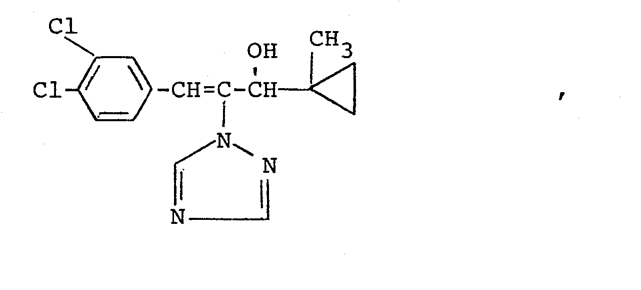 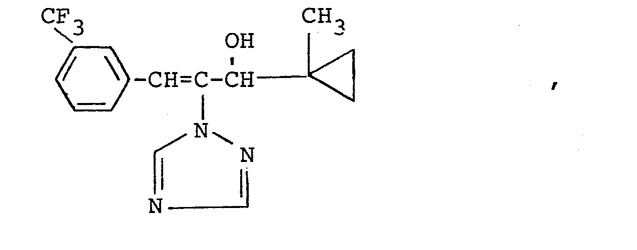 und 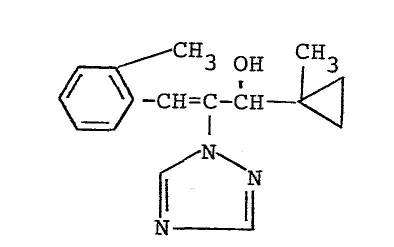 gefunden.

Die erfindungsgemäßen Verbindungen kommen in den geometrischen Isomeren E (trans) und Z (cis) vor.

Bei der E,Z-Nomenklatur werden die an der Doppelbindung stehenden Substituenten nach der Cahn-Ingold-Prelog-Regel nach abnehmender Priorität eingeordnet. Stehen die bevorzugten Substituenten auf derselben Seite der Doppelbindung, liegt die Konfiguration Z (abgeleitet von zusammen) vor, stehen sie auf entgegengesetzter Seite, liegt die Konfiguration E (abgeleitet von entgegen) vor.

Da außerdem ein asymmetrisches Kohlenstoffatom vorhanden ist, können die erfindungsgemäßen Triazolylpropenol-Derivate in zwei optischen Isomerenformen vorkommen.

Die vorliegende Erfindung betrifft sowohl die einzelnen Isomeren als auch die Isomerengemische.

Weiterhin wurde gefunden, daß man die erfindungsgemäßen Triazolylpropenol-Derivate erhält, wenn man Triazolylpropenon-Derivate der Formeln 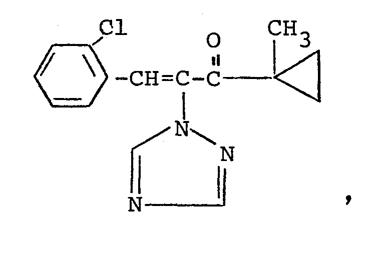 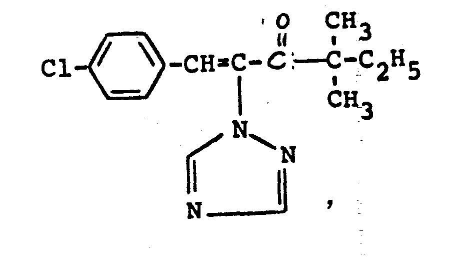 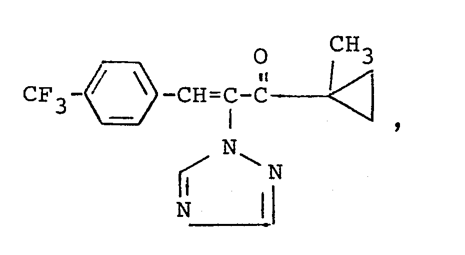 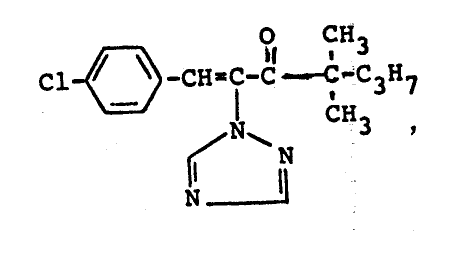 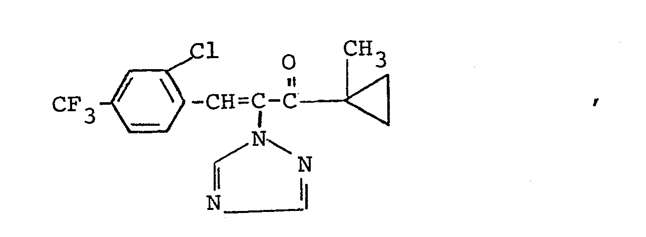 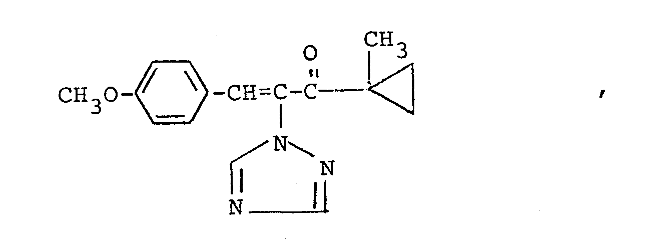 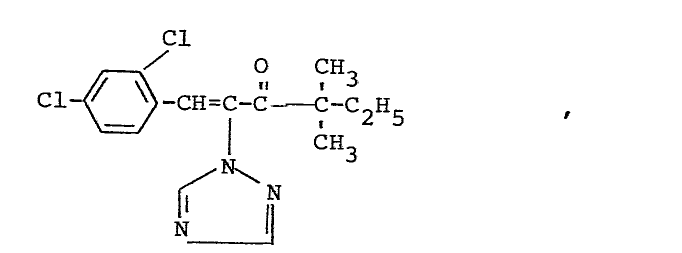 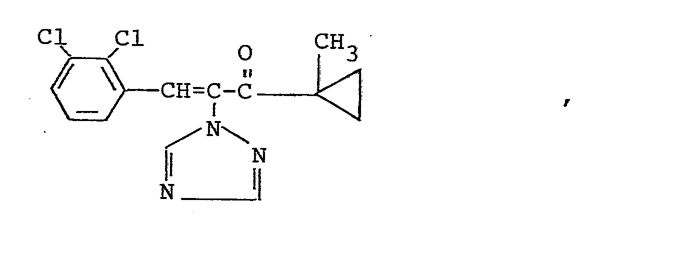 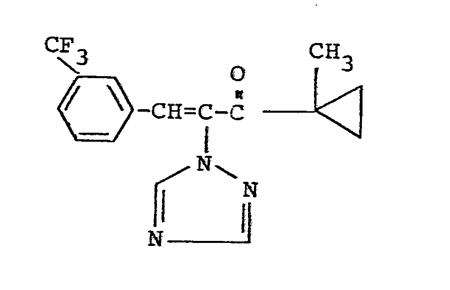 und 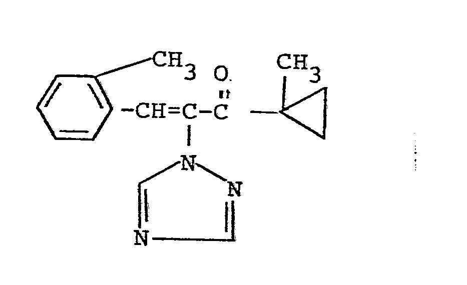 in allgemein üblicher Weise reduziert. Schließlich wurde gefunden, daß die neuen Triazolylpropenol-Derivate starke pflanzenwachstumsregulierende und starke fungizide Eigenschaften aufweisen.

überraschenderweise zeigen die erfindungsgemäßen Triazolylpropenol-Derivate eine bessere wachstumsregulierende und fungizide Wirkung als die aus dem Stand der Technik bekannten 4,4-Dimethyl-1-phenyl-2-triazolyl-1-penten-3-ole, welche chemisch und wirkungsmäßig naheliegende Verbindungen sind. Die erfindungsgemäßen Wirkstoffe stellen somit eine Bereicherung der Technik dar.

Verwendet man bei der erfindungsgemäßen Reduktion beispielsweise 1-Methylcycloprop-1-yl-[1-(1,2,4-triazol-1-yl)₋2-(2-chlorphenyl)-ethen-1-yl/-keton und Aluminiumisopropylat als Ausgangsstoffe, so kann der Reaktionsablauf durch das folgende Formelschema wiedergegeben werden:

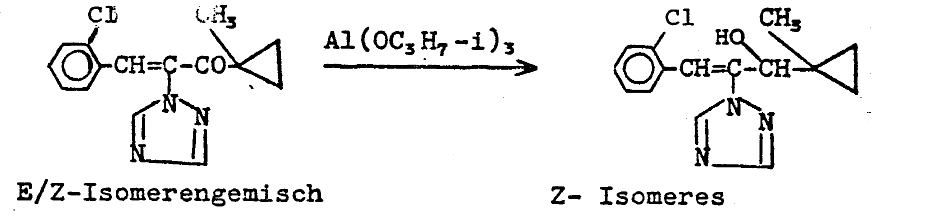

Die bei der Durchführung der erfindungsgemäßen Reduktion als Ausgangsstoffe benötigten Triazolylpropenon-Derivate sind noch nicht bekannt. Sie können jedoch in bekannter Art und Weise erhalten werden, indem man Triazolylketone der Formel 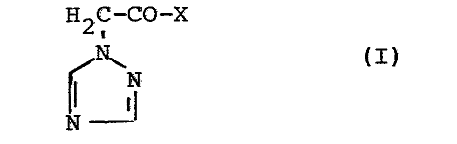 in welcher 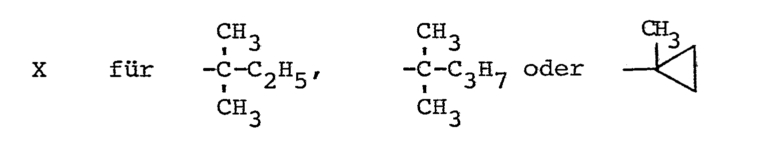 steht, mit Aldehyden der Formeln 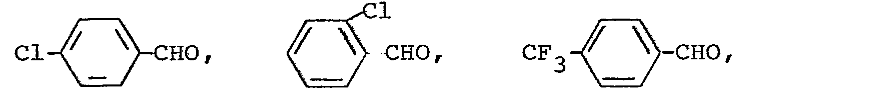 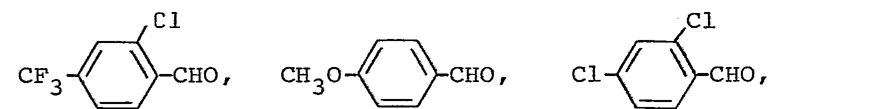 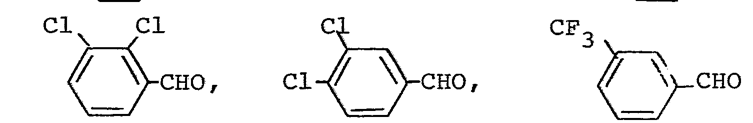 oder 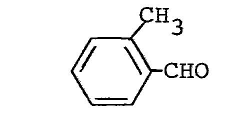 in Gegenwart eines Lösungsmittels und in Gegenwart eines Katalysators umsetzt.

Als Lösungsmittel kommen für die Herstellung der Triazolylpropenonderivate vorzugsweise inerte organische Lösungsmittel in Frage. Hierzu gehören vorzugsweise Alkohole, wie Methanol und Ethanol; Ether, wie Tetrahydrofuran und Dioxan; aliphatische und cycloaliphatische Kohlenwasserstoffe, wie Hexan und Cyclohexan; aromatische Kohlenwasserstoffe, wie Benzol, Toluol und Cumol; halogenierte aliphatische und aromatische Kohlenwasserstoffe, wie Methylenchlorid, Tetrachlorkohlenstoff, Chloroform, Chlorbenzol und Dichlorbenzol.

Die Herstellung der Triazolylpropenon-Derivate wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren, wie z.B. Bortrifluorid, Bortrichlorid, Zinntetrachlorid oder Titantetrachlorid; organische Basen wie Pyridin und Piperidin; sowie insbesondere Piperidinacetat.

Die Reaktionstemperaturen können bei der Durchführung dieses Verfahrens in einem größeren Bereich variiert werden. Im allgemeinen arbeitet man zwischen 20 und 160°C, vorzugsweise bei der Siedetemperatur des jeweiligen Lösungsmittels.

Bei der Durchführung dieses Verfahrens setzt man auf 1 Mol Triazolylketon der Formel (I) 1 bis 1,5 Mol Aldehyd und katalytische bis 0,2 molare Mengen an Katalysator ein. Die Triazolylpropenon-Derivate fallen vorzugsweise als E/Z-Isomerengemische an. Eine Trennung in die reinen Isomeren ist auf übliche Art und Weise möglich, wie z.B. durch Kristallisation oder durch chromatographische Trennverfahren.

Die Triazolylpropenon-Derivate stellen allgemein interessante Zwischenprodukte dar, wie z.B. zur Herstellung der erfindungsgemäßen Triazolylpropenol-Derivate. In entsprechenden Konzentrationen zeigen sie auch wachstumsregulierende und fungizide Eigenschaften.

Die Triazolylketone der Formel (I) sind teilweise bekannt (vgl. DE-OS 24 31 407 und DE-A 26 38 470).

Die Triazolylketone der Formel (I) können nach allgemein bekannten Verfahren erhalten werden, indem man Halogenketone der Formel 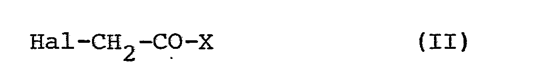 in welcher X die oben angegebene Bedeutung hat und

Hal für Chlor oder Brom steht, mit 1,2,4-Triazol in Gegenwart eines Verdünnungsmittels und in Gegenwart eines Säurebindemittels umsetzt.

Als Verdünnungsmittel kommen für die Herstellung der Triazolylketone der Formel (I) vorzugsweise inerte organische Lösungsmittel in Frage. Hierzu gehören vorzugsweise Ketone, wie Aceton und Methylethylketon; Nitrile, wie Acetonitril; Ether, wie Tetrahydrofuran oder Dioxan; aromatische Kohlenwasserstoffe, wie Benzol und Toluol; Formamide, wie Dimethylformamid; und halogenierte Kohlenwasserstoffe.

Dieses Verfahren wird in Gegenwart eines Säurebinders vorgenommen. Man kann alle üblicherweise verwendbaren anorganischen oder organischen Säurebinder zugeben, wie Alkalicarbonate, b ispielsweise Natriumcarbonat, Kaliumcarbonat und Natriumhydrogencarbonat; Alkalialkoholate, wie Natriummethylat oder Natriumethylat; niedere tertiäre Alkylamine, Cycloalkylamine oder Aralkylamine, wie Triethylamin, Dimethylbenzylamin oder Dimethylcyclohexylamin; oder wie Pyridin und ein entsprechender überschuß an 1,2,4-Triazol.

Die Reaktionstemperaturen können bei der Durchführung dieses Verfahrens in einem größeren Bereich variiert werden. Im allgemeinen arbeitet man zwischen 0 bis 120°C, vorzugsweise zwischen 20 und 100°C.

Bei der Durchführung dieses Verfahrens setzt man auf 1 Mol der Verbindungen der Formel (II) vorzugsweise 1 bis 2 Mol 1,2,4-Triazol und 1 bis 2 Mol Säurebinder ein. Die Isolierung der Verbindungen der Formel (I) erfolgt in üblicher Weise.

Die Halogenketone der Formel (II) sind bekannt bzw. können sie in allgemein bekannter Art und Weise erhalten werden, indem man Verbindungen der Formel 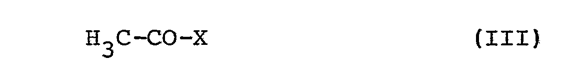 in welcher X die oben angegebene Bedeutung hat, in Gegenwart eines inerten organischen Lösungsmittels bei Raumtemperatur mit Chlor oder Brom versetzt; oder z.B. mit üblichen Chlorierungsmitteln, wie Sulfurylchlorid bei 20 bis 60°C umsetzt.

Die außerdem für die Herstellung der Triazolylpropenon-Derivate als Ausgangsstoffe benötigten Aldehyde sind allgemein bekannte Verbindungen der organischen Chemie.

Die erfindungsgemäße Reduktion erfolgt in üblicher Weise, z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels. Die Ausgangsstoffe können dabei als E/Z-Isomerengemische oder als reine Iso- meren eingesetzt werden.

Arbeitet man mit komplexen Hydriden, so kommen als Verdünnungsmittel für die erfindungsgemäße Umsetzung polare organische Lösungsmittel in Frage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei -10 bis +30°_{C}, vorzugsweise bei -10 bis 20°C durchgeführt. Hierzu setzt man auf 1 Mol Triazolylpropenon-Derivat etwa 1 Mol eines komplexen Hydrids, wie Natriumborhydrid, Calciumborhydrid oder Lithiumalanat, ein. Die Isolierung der erfindungsgemäßen Verbindungen erfolgt in üblicher Weise, ebenso eine eventuelle Trennung der E/Z-Isomerengemische, die bei der Reduktion mit kom₋ plexen Hydriden immer entstehen, wenn man von E/Z-Isomerengemischen als Ausgangsprodukten ausgeht.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, in Frage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des entsprechenden Triazolylpropenon-Derivates etwa 1 bis 2 Mol Aluminiumisopropylat ein. Die Isolierung der erfindungsgemäßen Verbindungen erfolgt in üblicher Weise.

Bei der Reduktion mit Aluminiumisopropylat erhält man ausschließlich die Z-Isomeren.

Ein eindeutiges Charakterisierungsmerkmal für die beiden geometrischen Isomeren ist die H¹⁻Kernresonanz der zwei Triazol-Protonen. Die Differenz der Shiftwerte für diese beiden Protonen ist in den E-Formen ungefähr doppelt so groß wie in den entsprechenden Z-Formen.

Die erfindungsgemäß verwendbaren Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können z.B. zur Hemmung des vegetativen Wachstums der Pflanzen eingesetzt werden. Eine derartige Wuchshemmung ist u.a. bei Gräsern von wirtschaftlichem Interesse, denn dadurch kann die Häufigkeit der Grasschnitte in Ziergärten, Park-und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch, daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldomianz die Entwicklung von Seitentrieben gefördert werden,.was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jah sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständig mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die erfindungsgemäßen Wirkstoffe weisen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Al Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z.B. gegen den Erreger des Gersten- bzw. des Getreidemehltaus (Erysiphe graminis), oder zur Bekämpfung von Podosphaera-Arten, wie z.B. gegen den Erreger des Apfelmehltaus (Podosphaera leucotricha). Außerdem zeigen die erfindungsgemäßen Stoffe ein breites fungizides in-vitro-Spektrum.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark plare Lösungsmittel, wie Dimethylformamid und Dimethyl:.:ulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase,wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie

Polyoxyethylen-Fettsäureester, Polyoxyethylen-Fettalkoholether, z.B. Alkylarylpolyglykol-ether, Alkylsulfonate, Alkylsulfate, Arylsulfonate sowie Eiweißhydrolysate. Als Dispergiermitt l kommen in Frage:
z.B. Lignin-Sulfitablaugen und Methylcellulose. Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo―und Metallphthalocyaninfarbstoffeund Spurennährstoffe, wie Salze und Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen in Mischung mit anderen Wirkstoffen vorliegen, wie Fungizide, Insektizide, Akarizide und Herbizide, sowie in Mischung mit Düngemitteln und anderen Wachstumsregulatoren.

Die Wirkstoffe können als solche, in Form ihrer Formulierungen oder der daraus bereiteten Anwendungsfor-en, wie gebrauchsfertige Lösungen, emulgierbare Konzent ate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,''5 bis 10 kg.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Auch beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50 g ie kq Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

### Herstellungsbeispiele

### Beispiel 1

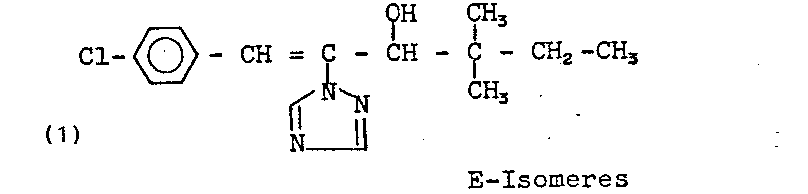

2,88 g (9,5 Mol) E-Isomeres 1-(4-Chlorphenyl)-4,4-dimethyl-2-(1,2,4-triazol-1-yl)-1-hexen-3-on werden in 20 ml Isopropanol gelöst und mit 180 mg (4,75 mMol) Natriumboranat versetzt. Nach 15-stündigem Rühren bei Raumtemperatur wird das Isopropanol im Vakuum abdestilliert und der Rückstand mit Wasser und Eisessig zersetzt. Die organische Phase wird abgetrennt, in Methylenchlorid gelöst, mit Wasser gewaschen, über Natriumsulfat getrocknet und eingedampft. Das resultierende öl wird mit Diisopropylether verrührt, die entstehenden Kristalle werden abgesaugt und getrocknet. Man erhält 700 mg (24 % der Theorie) E-Isomeres 1-(4-Chlorphenyl)-4,4-dimethyl-2-(1,2,4-triazol-1-yl)-1-Hexen-3-ol vom Schmelzpunkt 130°C.

### Herstellung des Ausgangsproduktes

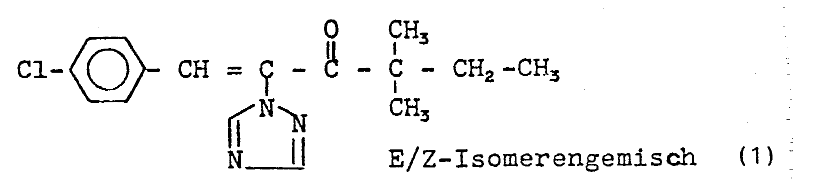

und 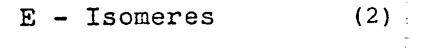 81,5 g (0,45 Mol) 3,3-Dimethyl-1-(1,2,4-triazol-1-yl)-pentan-2-on und 63,2 g (0,45 Mol) 4-Chlorbenzaldehyd werden in 500 ml Toluol mit 12,5 ml Essigsäure und 4,5 ml Piperidin 15 Stunden unter Rückfluß erhitzt und das Reaktionswasser azeotrop entfernt. Die Toluollösung wird mit Wasser gewaschen, über Natriumsulfat getrocknet und im Vakuum eingedampft. Man erhält 127 g (93 % der Theorie) 1-(4-Chlorphenyl)-4,4-dimethyl-2-(1,2,4-triazol-1-yl)-1-hexen-3-on als E/Z-Isomerengemisch vom Siedepunkt 150 bis 160°C/0,1 Torr.

Nach mehrtägigem Stehen bei Raumtemperatur kristallisiert E-Isomeres 1-(4-Chlorphenyl)-4,4-dimethyl-2-(1,2,4-triazol-1-yl)-1-hexen-3-on vom Schmelzpunkt 90°C aus. 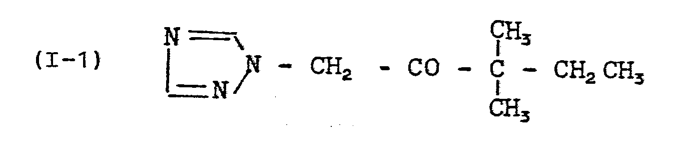 Zu einem Gemisch von 62,1 g (0,9 Mol) 1,2,4-Triazol, 95,4 g (0,69 Mol) Kaliumcarbonat und 600 ml Aceton werden bei 55°C 106 g (0,55 Mol) 1-Brom-3,3-dimethylpentan-2-on eingetropft. Nach 15 Stunden Rühren wird das Gemisch filtriert und das Filtrat im Vakuum eingedampft. Das verbleibende öl wird chromatographisch gereinigt (Kieselgel-60(Merck)/Chloroform).

### Man erhält 85,6 g (86 % der Theorie) 3,3-Dimethyl-1-(1,2,4-triazol-1-yl)-pentan-2-on vom Brechungsindex n_{D} = 1,4805.

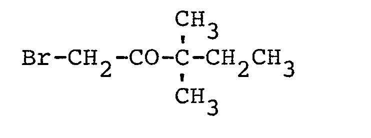

Zu einer Lösung von 69 g (0,6 Mol) 3,3-Dimethylpentan-2 on in 300 ml Methylalkohol wird bei 0 bis 5°C eine Lösung von 30,6 g (0,6 Mol) Brom in 120 ml Chloroform getropft und 15 Minuten nachgerührt. Die Reaktionsmi chung wird auf Eis gegeben, die organische Phase abgetrennt, mit Wasser gewaschen, über Natriumsulfat getrocknet, filtriert und eingedampft. Das Rohprodukt wird im Vakuum destilliert. Man erhält 101 g (87 % der Theorie) 1-Brom-3,3-dimethylpentan-2-on vom Siedepunkt 80 bis 88°C/11 Torr bzw. vom Brechungsindex nD = 1,468₅.

### Beispiel 2

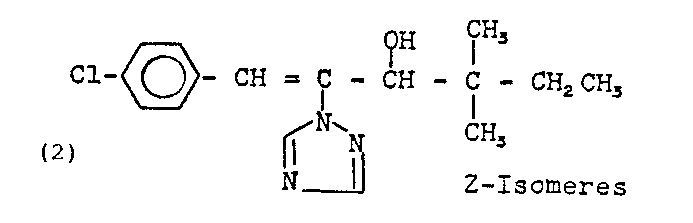

30,35 g (0,1 Mol) 1-(4-Chlorphenyl)-4,4-dimethyl-2-(1,2,4-triazol-1-yl)-1-hexen-3-on als E/Z-Isomerengemisch (vgl. Herstellung des Ausgangsproduktes Beispiel 1) werden entsprechend Beispiel 1 mit Natriumboranat reduziert. Das erhaltene Öl (30,5 g) wird an Kieselgel-60(Merck)/Chloroform chromatographiert. Die nach Abdampfen des Chloroforms erhaltenen Fraktionen vom Schmelzpunkt 100 bis 108°C werden vereinigt und zweimal aus Acetonitril umkristallisiert. Man erhält Z-Isomeres 1-(4-Chlorphenyl)-4,4-dimethyl-2-(1,2,4-triazol-1-yl)-1-hexen-3-ol vom Schmelzpunkt 119°C.

In entsprechender Weise werden die in der folgenden Tabelle 1 aufgeführten Verbindungen hergestellt.

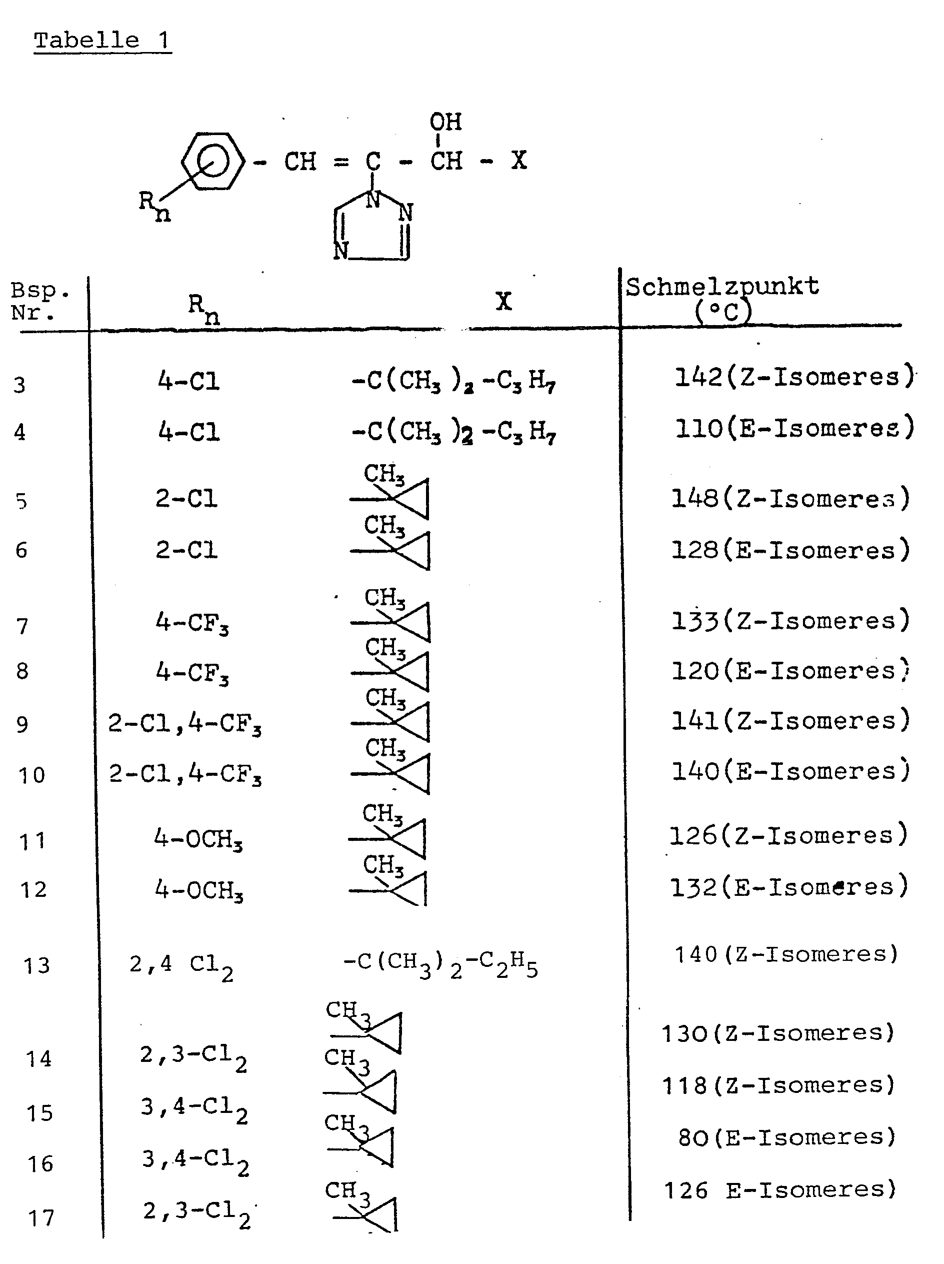

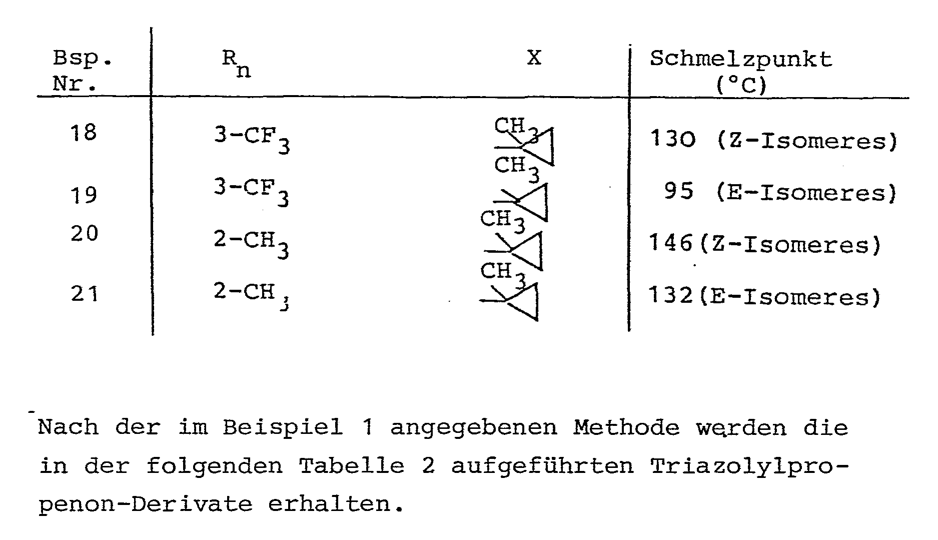

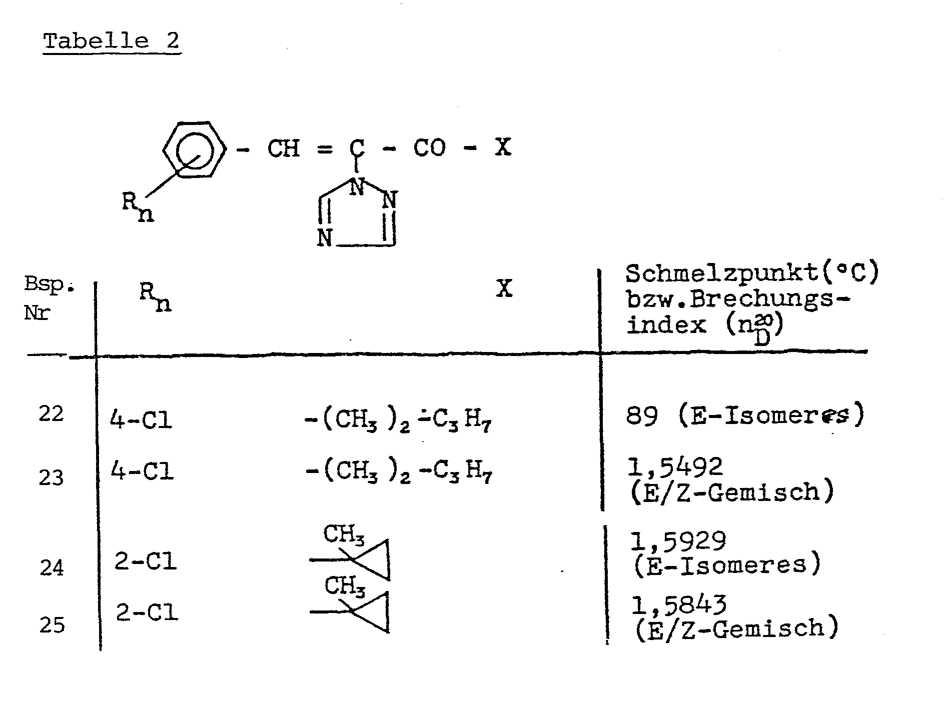

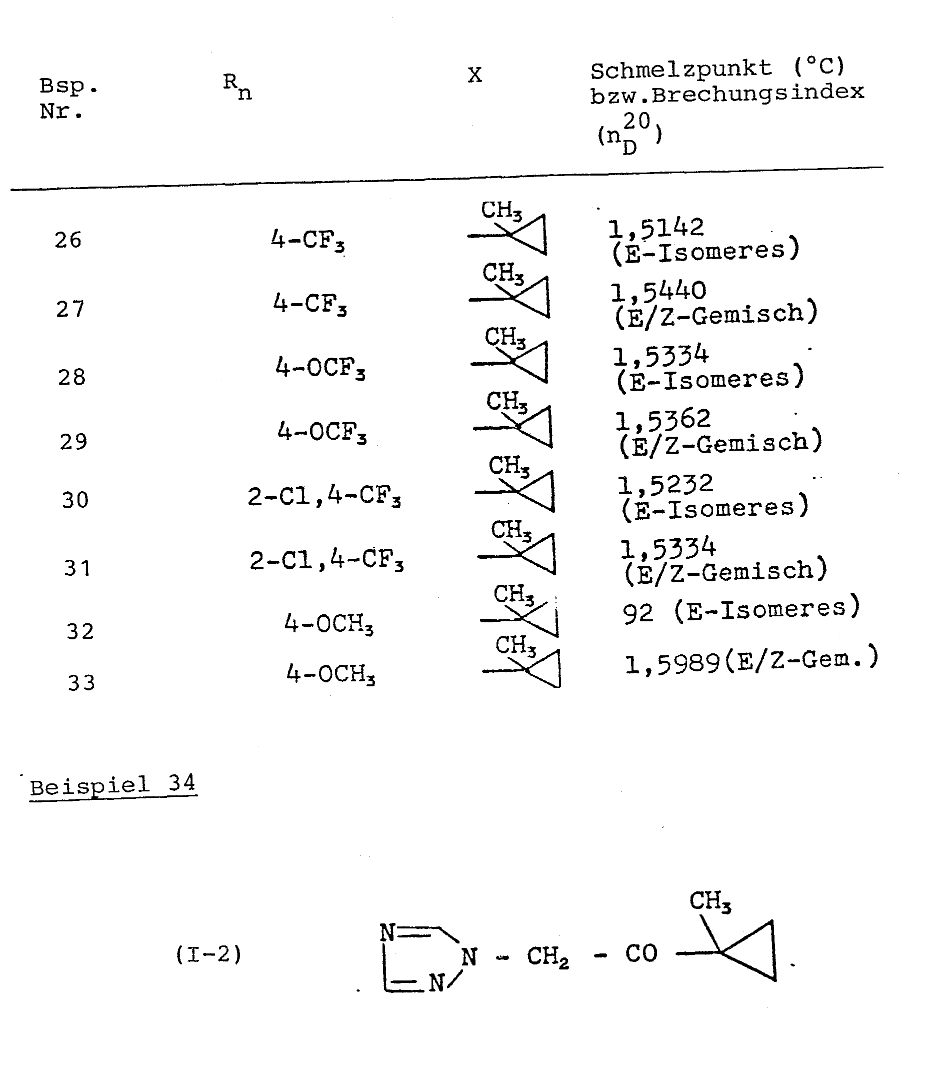

In eine Suspension von 27,6 g (0,4 Mol) 1,2,4-Triazol und 41,4 g (0,3 Mol) Kaliumcarbonat in 500 ml Aceton werden bei 60°C 42,5 g (0,24 Mol) 1-Bromacetyl-1-methylcyclopropan eingetropft. Nach 15 Stunden Erhitzen auf 60°C werden die Salze abgesaugt und das Filtrat im Vakuum eingedampft. Das verbleibende öl wird chromatographisch (Kieselgel-60(Merck)/Chloroform) gereinigt.

Man erhält 35,7 g (90 % der Theorie) 1-Methyl-1-(1,2,4-triazol-1-yl-acetyl)-cyclopropan vom Schmelzpunkt 58°C.

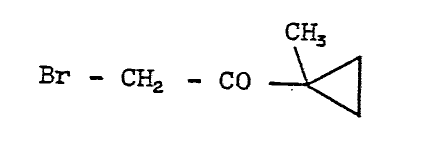

Zu einer Lösung von 29,4 g (0,3 Mol) 1-Acetyl-1-methyl- cyclopropan in 150 ml Methylalkohol werden bei 5°C 15 ml Brom, gelöst in 75 ml Chloroform, getropft.

Die Lösung wird bei 10°C bis zur vollständigen Entfärbung gerührt, auf Eis gegeben und mit Wasser gewaschen. Die Chloroformphase wird über Natriumsulfat getrocknet, filtriert, eingedampft und destilliert.

Man erhält 44 g (82,5 % der Theorie) 1-Bromacetyl-1-methylcyclopropan vom Siedepunkt 85 bis 90°C/11 Torr bzw. vom Brechungsindex 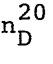 = 1,5002.

### Beispiel 35

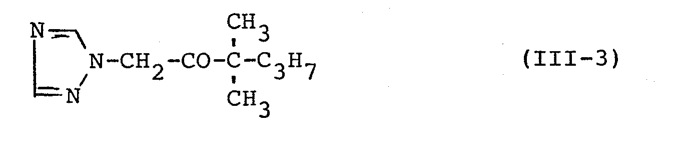

Die Herstellung erfolgt nach der Methode, die in den entsprechenden vorhergehenden Beispielen beschrieben ist. Brechungsindex:

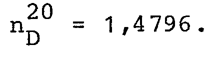

### Anwendungsbeispiele

In den nachfolgenden Anwendungsbeispielen werden die nachstehend angegebenen Verbindungen als Vergleichssubstanzen eingesetzt:

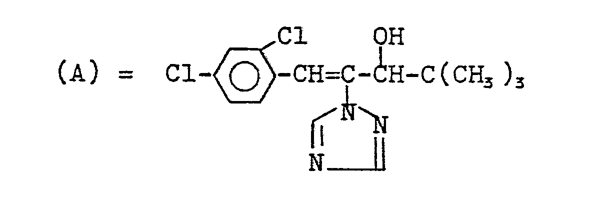

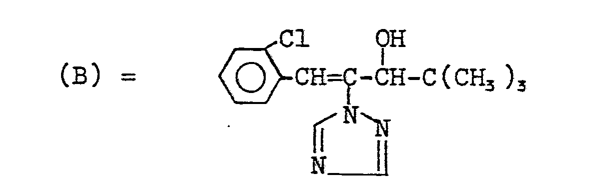

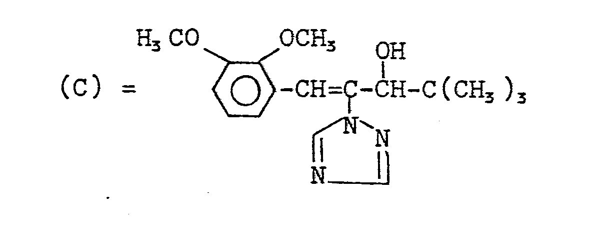

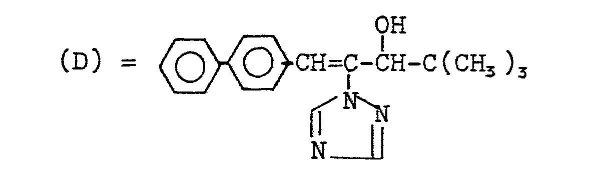

### Beispiel A

### Wuchshemmung bei Gras (Festuca pratensi.s)

Lösungsmittel: 30 Gew.-Teile Dimethylformamid Emulgator: 1 Gew.-Teil Polyoxyethylen-SorbitanMonolaurat

Zur Herstellung einer zweckmäßigen Wirkstoffzubereitung vermischt man 1 Gew.-Teil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnaß besprüht. Nach 3 Wochen wird der Zuwachs gemessen und die Wuchshemmung in Prozent des Zuwachses der Kontrollpflanzen berechnet. Es bedeuten 100 % Wuchshemmung den Stillstand des Wachstumes und 0 % ein Wachstum entsprechend dem der Kontrollpflanzen.

Der Wirkstoff gemäß Beispiel 19 zeigt in diesem Test eine bessere Wuchshemmung als die aus dem Stand der Technik bekannten Verbindungen (A), (B), (C) und (D).

### Beispiel B

### Wuchshemmung bei Baumwolle

Lösungsmittel: 30 Gew.-Teile Dimethylformamid Emulgator: 1 Gew.-Teil Polyoxyethylen-SorbitanMonolaurat

Zur Herstellung einer zweckmäßigen Wirkstoffzubereitung vermischt man 1 Gew.-Teil Wirkstoff mit der angegebenen Menge Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Die Wirkstoffe gemäß Beispielen 1,2, 15, 17 und 19 zeigen in diesem Test eine bessere Wuchshemmung als die aus dem Stand der Technik bekannten Verbindungen (A), (B), (C) und (D).

### Beispiel C

### Wuchshemmung bei Sojabohnen

Lösungsmittel: 10 Gew.-Teile Methanol Emulgator: 2 Gew.-Teile Polyethylen-SorbitanMonolaurat

Zur Herstellung einer zweckmäßigen Wirkstoffzubereitung vermischt man 1 Gew.-Teil Wirkstoff mit den angegebenen Mengen Lösungsmittel und Emulgator und füllt mit Wasser auf die gewünschte Konzentration auf.

Junge Sojabohnenpflanzen werden in dem Stadium, in dem die ersten Folgeblätter entfaltet sind, mit den Wirkstoffzubereitungen tropfnaß besprüht. Nach 2 Wochen wird der Zuwachs gemessen und die Wuchshemmung in % des Zuwachses der Kontrollpflanzen berechnet. Es bedeuten 100 % den Stillstand des Wachstums und 0 % ein Wachstum entsprechend dem der unbehandelten Kontrollpflanzen.

Die Wirkstoffe gemäß Beispielen 1, 15 und 17 zeigen in diesem Test eine bessere Wuchshemmung als die aus dem Stand der Technik bekannten Verbindungen (B), (C) und (D).

### Beispiel D

### Wuchsbeeinflussung bei Zuckerrüben

Lösungsmittel: 30 Gew.-Teile Dimethylformamid Emulgator: 1 Gew.-Teil Polyoxyethylen-SorbitanMonolaurat

Zur Herstellung einer zweckmäßigen Wirkstoffzubereitung vermischt man 1 Gew.-Teil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit der Wirkstoffzubereitung besprüht. Nach 14 Tagen wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend dem der Kontrollpflanzen. Negative Werte kennzeichnen eine Wuchshemmung, positive Werte eine Wuchsförderung gegenüber den Kontrollpflanzen.

Die Wirkstoffe gemäß Beispielen 1, 2, 15 und 17 zeigen in diesem Test eine bessere Wuchsbeeinflussung als die aus dem Stand der Technik bekannten Verbindungen (C) und (D).

### Beispiel E

Erysiphe-Test (Gerste) /protektiv/

Lösungsmittel: 100 Gew.-Teile Dimethylformamid Emulgator: 0,25 Gew.-Teile Alkyl-aryl-polyglykolether

Zur Herstellung einer zweckmäßigen Wirkstoffzubereitung venischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z.B. die Verbindungen gemäß folgender Herstellungsbeispiele:
1 und 2.

## Patentansprüche

1. Triazolylpropenol-Derivate der Formeln

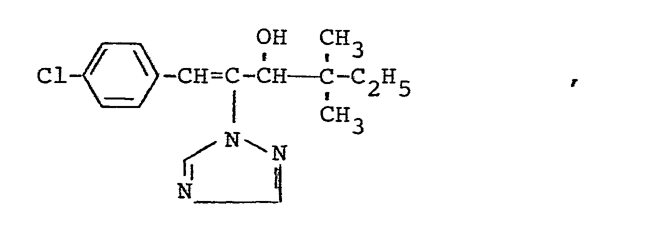

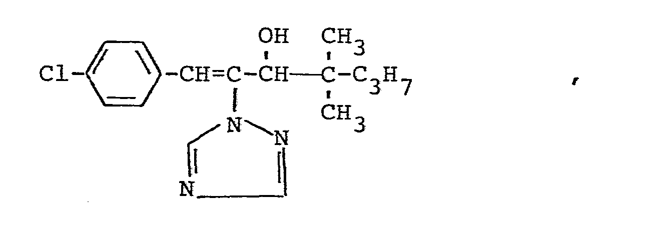

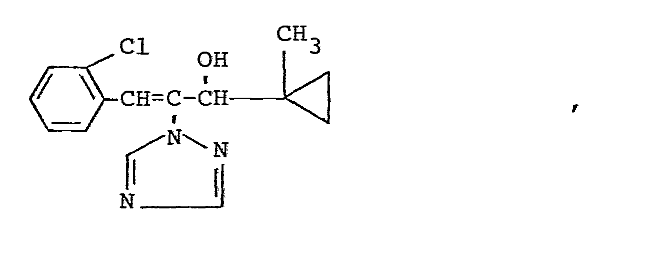

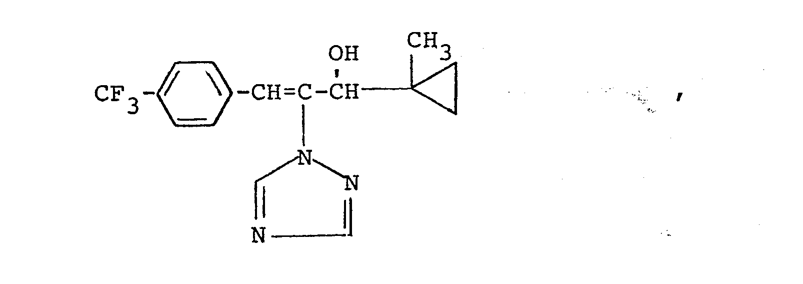

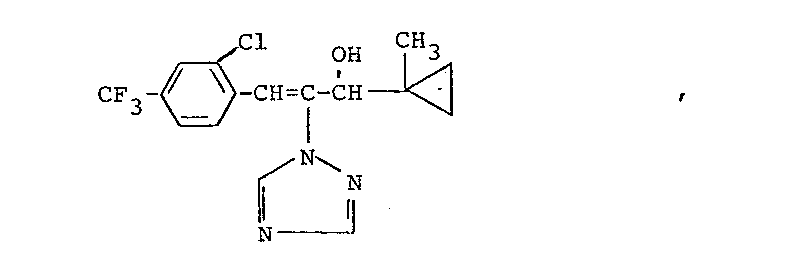

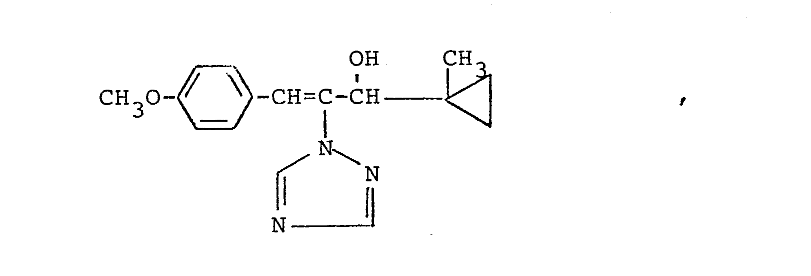

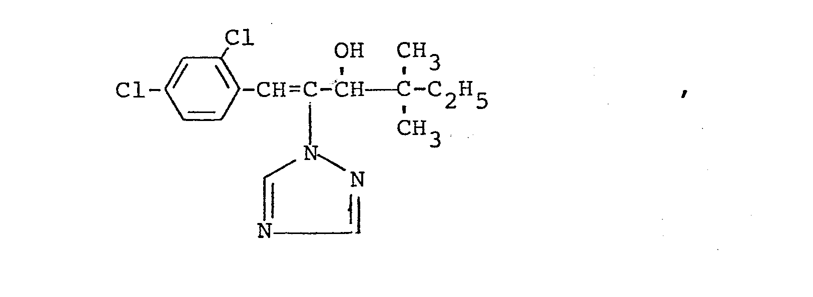

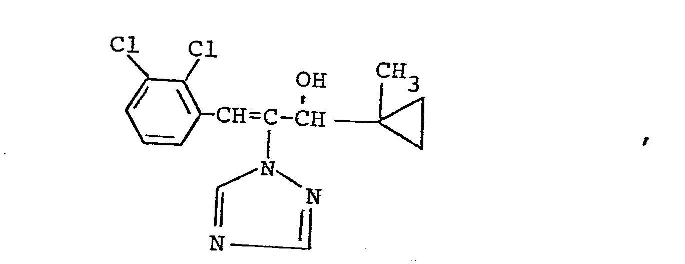

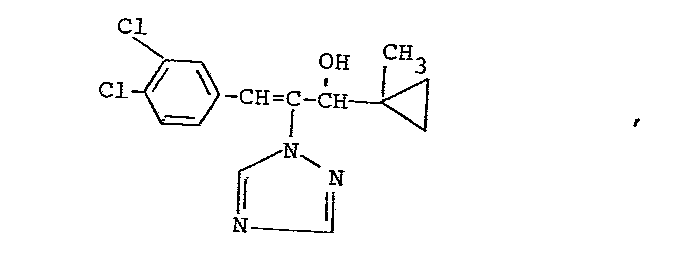

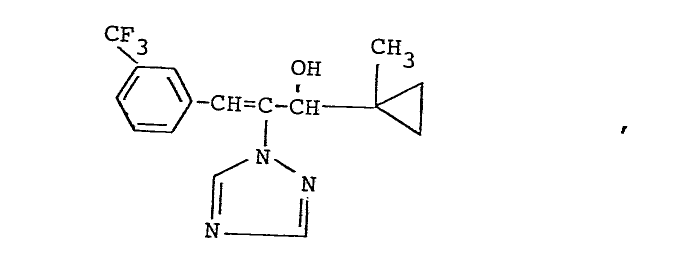

und

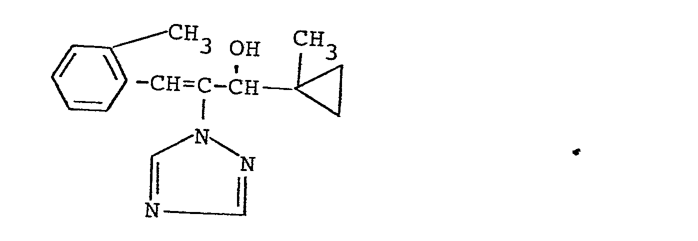

2. Verfahren zur Herstellung von Triazolyl-propenol-Derivaten der Formeln 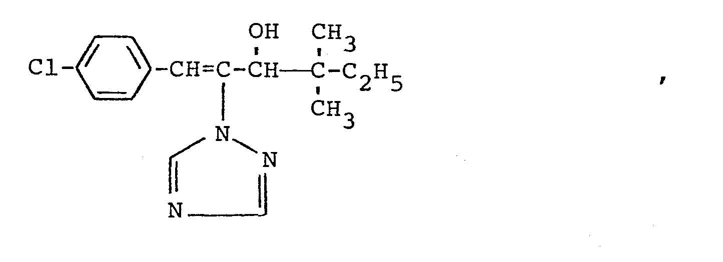 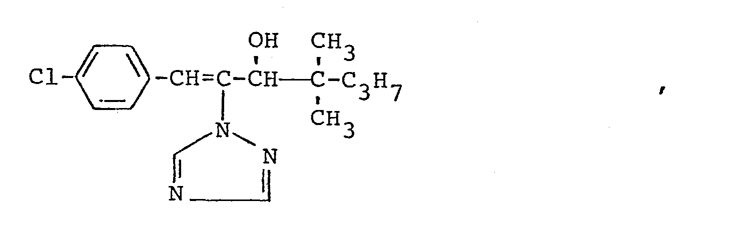 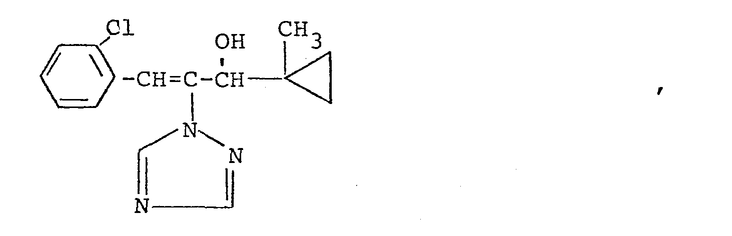 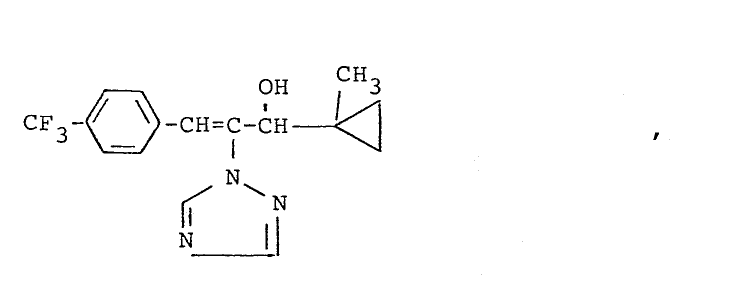 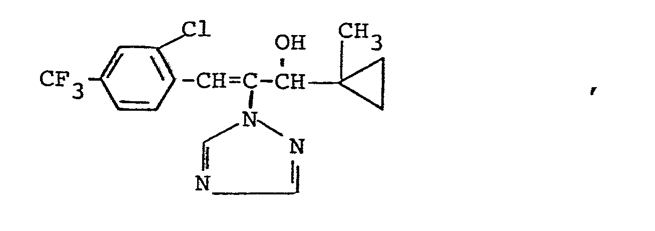 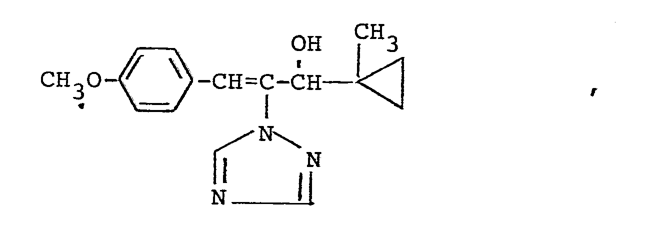 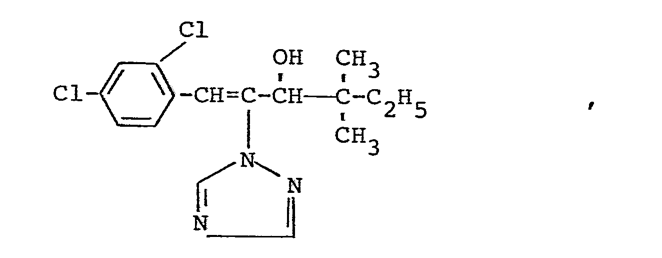 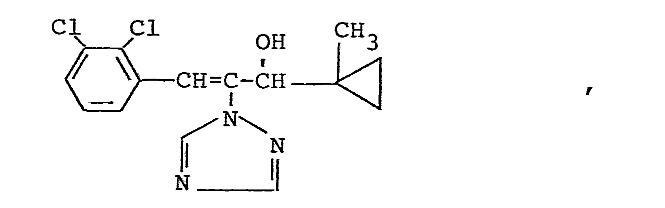 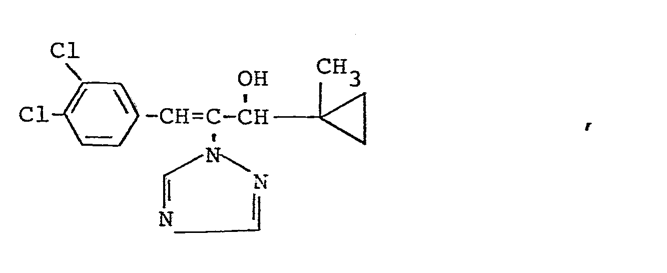 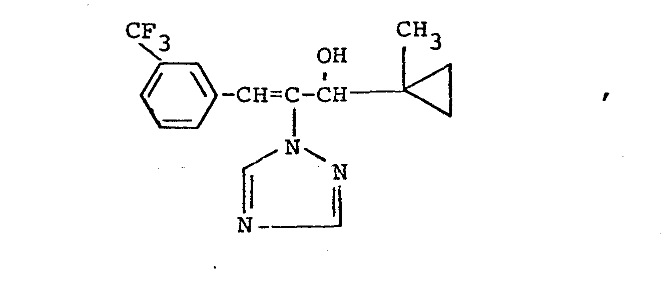 und 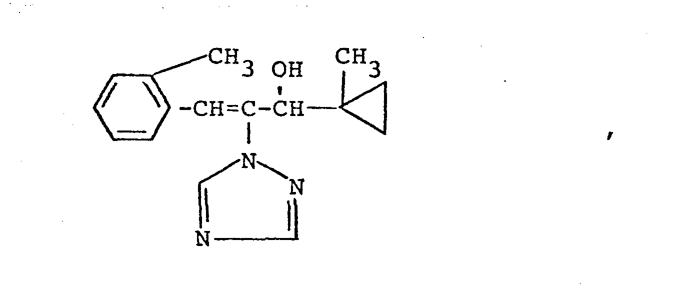 dadurch gekennzeichnet, daß man Triazolylpropenon-Derivate der Formeln 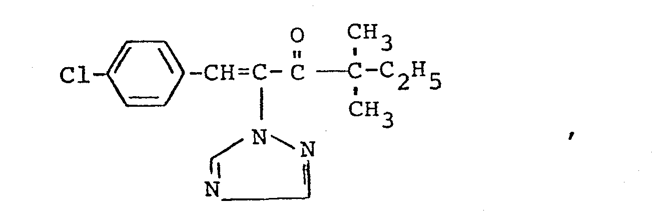 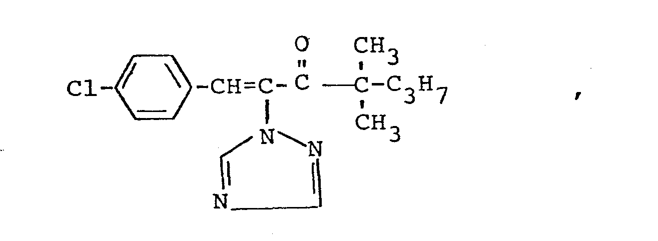 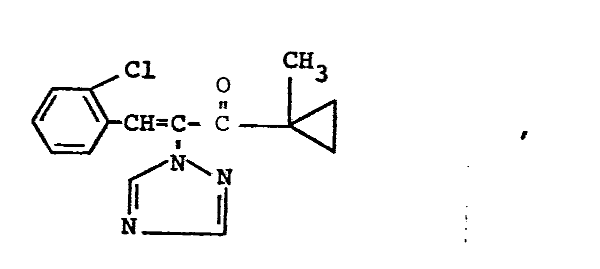 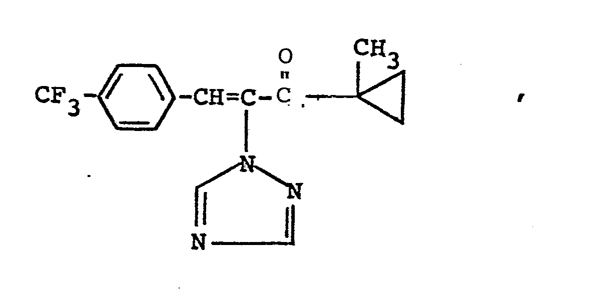 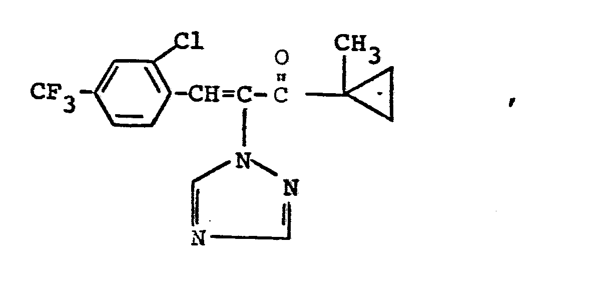 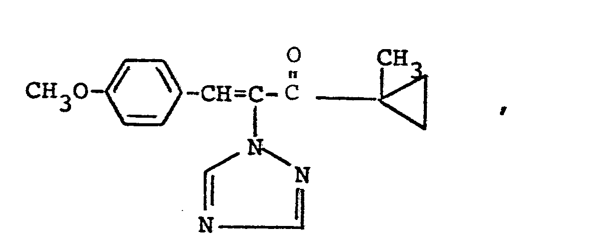 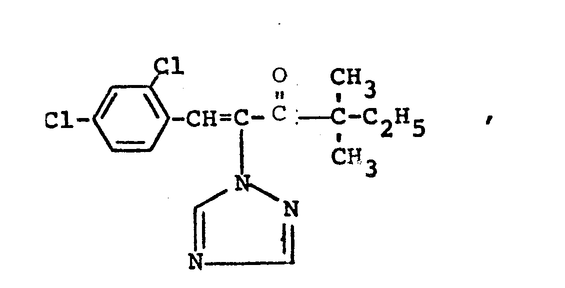 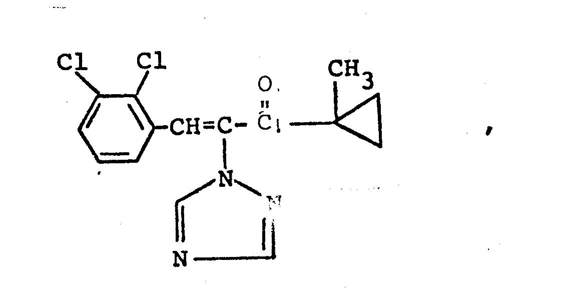 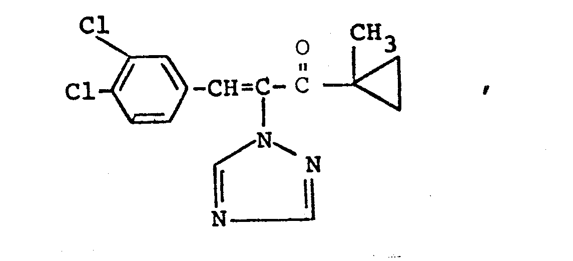 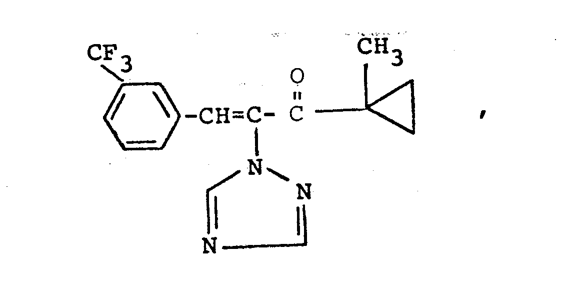 und 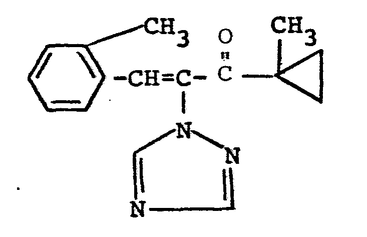 in allgemein üblicher Weise reduziert.

3. Pflanzenwachstumsregulierende und fungizide Mittel, gekennzeichnet durch einen Gehalt an mindestens einem Triazolylpropenol-Derivat gemäß Anspruch 1.

4. Verfahren zur Regulierung des Pflanzenwachstums und zur Bekämpfung von Pilzen, dadurch gekennzeichnet, daß man Triazolylpropenol-Derivate gemäß Anspruch 1 auf die behandelnden Pflanzen -der ihren Lebensraum einwirken läßt.

5. Verwendung von Triazolylpropenol-Derivaten gemäß Anspruch 1 zur Regulierung des Pflanzenwachstums und zur Bekämpfung von Pilzen.

6. Verfahren zur Herstellung von pflanzenwachstumsregulierenden und fungiziden Mitteln, dadurch gekennzeichnet, daß man Triazolylpropenol-Derivate gemäß Anspruch 1 mit Streckmitteln und/oder oberflächenaktiven Mitteln vermischt.

7. Triazolylpropenon-Derivate der Formeln 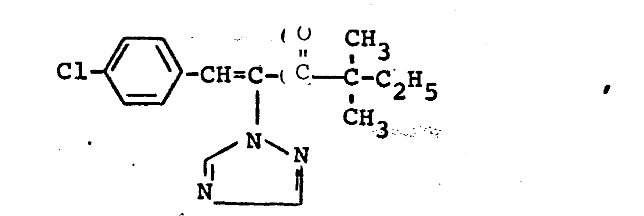 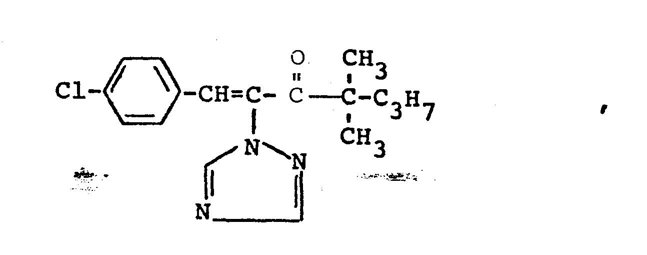 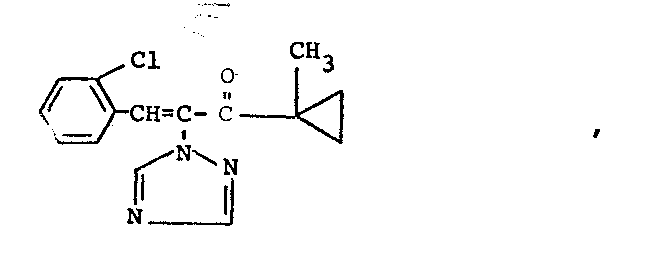 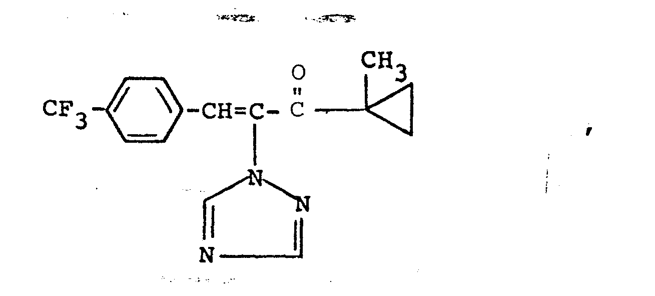 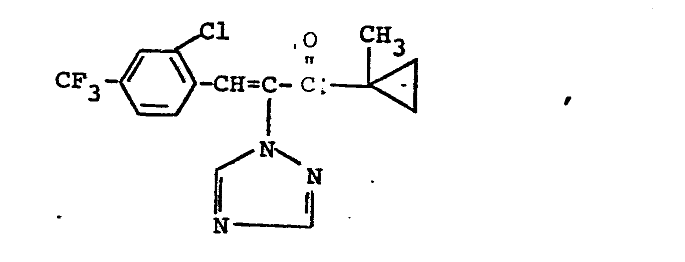 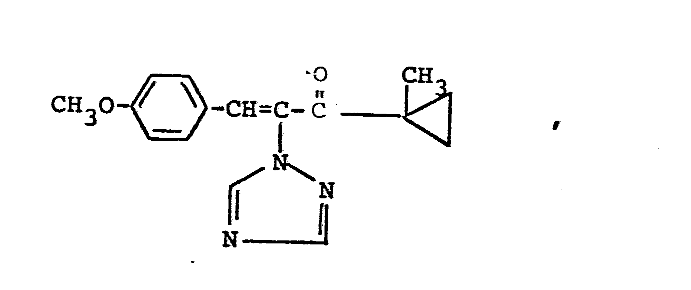 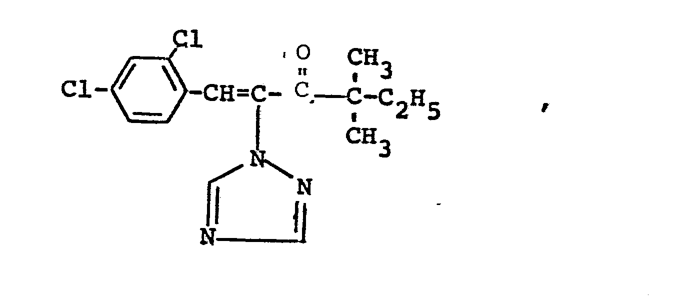 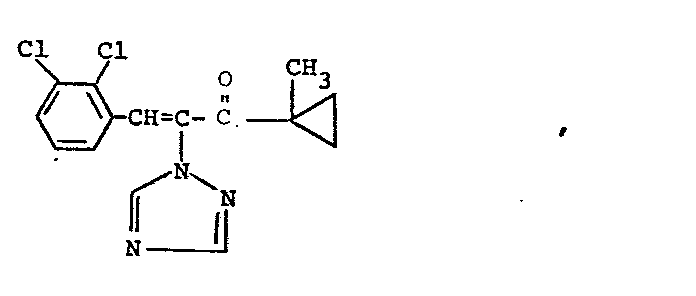 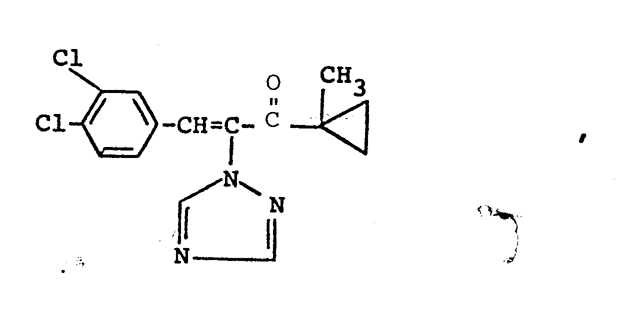 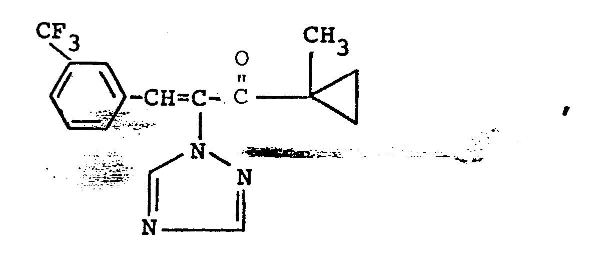 und
